(19) **Europäisches Patentamt**

**European Patent Office**

**Office européen des brevets**

(11) **EP 1 187 586 B1**

(12) **EUROPÄISCHE PATENTSCHRIFT**

(45) Veröffentlichungstag und Bekanntmachung des Hinweises auf die Patenterteilung:
**07.09.2005 Patentblatt 2005/36**

(21) Anmeldenummer: **00935071.1**

(22) Anmeldetag: **19.05.2000**

(51) Int Cl.⁷: **A61F 13/02**

(86) Internationale Anmeldenummer:
**PCT/EP2000/004530**

(87) Internationale Veröffentlichungsnummer:
**WO 2000/074617 (14.12.2000 Gazette 2000/50)**

(54) **FILMPFLASTER UNTER VERWENDUNG VON TRÄGERFOLIEN MIT VERBESSERTEN GLEITEIGENSCHAFTEN UND GUTER DEHNBARKEIT, ERZIELT DURCH OPTIMIERUNG DER OBERFLÄCHENSTRUKTUR UND -HÄRTE**

FILM PLASTER USING SUPPORT FILMS WITH IMPROVED SLIDING PROPERTIES AND GOOD EXTENSIBILITY, ACHIEVED BY OPTIMISING THE SURFACE STRUCTURE AND HARDNESS

PANSEMENT PELLICULAIRE A BASE DE FEUILLES SUPPORT DOTEES DE CARACTERISTIQUES DE GLISSEMENT AMELIOREES ET D'UNE BONNE ELASTICITE, OBTENU PAR OPTIMISATION DE LA STRUCTURE ET DE LA DURETE SUPERFICIELLES

(84) Benannte Vertragsstaaten:
**AT BE CH CY DE DK ES FI FR GB GR IE IT LI LU MC NL PT SE**

(30) Priorität: **08.06.1999 DE 19925973**

(43) Veröffentlichungstag der Anmeldung:
**20.03.2002 Patentblatt 2002/12**

(73) Patentinhaber: **Beiersdorf AG**
**20245 Hamburg (DE)**

(72) Erfinder:
• **BRUSS, Witta**
**D-86199 Augsburg (DE)**
• **GÖTZ, Gabriela**
**D-22395 Hamburg (DE)**
• **MAYAN, Robert**
**D-21614 Buxtehude (DE)**

(56) Entgegenhaltungen:
**EP-A- 0 409 587      EP-A- 0 446 431**
**GB-A- 1 263 573      US-A- 4 439 391**
**US-A- 4 518 643      US-A- 5 328 450**
**US-A- 5 643 187**

EP 1 187 586 B1

**Beschreibung**

Die Erfindung betrifft Filmpflaster insbesondere zur Abdeckung von Wunden und Verhütung oder Behandlung von Blasen unter Verwendung von Trägerfolien mit definierten Oberflächeneigenschaften.

Filme und Folien finden in Pflastern und Wundschnellverbänden aufgrund ihrer Wasserundurchlässigkeit, ihrer Keimdichtigkeit, ihrer Anschmiegsamkeit und ihrer hohen Verträglichkeit häufig Verwendung.

So offenbart die DE 43 14 834 A1 ein Verband material auf Folienbasis, das auf der einen Seite mit einem Trägermaterial abgedeckt ist, das die gleiche Größe wie die Folie besitzt und mindestens eine Griffleiste aufweist, und das auf der anderen Seite mit einer selbstklebenden Schicht versehen ist. Erfindungswesentlich ist hier, daß die Griffleisten innerhalb der Umfassungsbegrenzung des Trägermaterials angeordnet sind. Vorzugsweise ist nur eine Griffleiste auf dem Trägermaterial angebracht.

Ein solches Pflaster mit Polyurethanfolie ist im Handel unter dem Namen "Aqua Protect" ® von der Firma Beiersdorf erhältlich.

Die DE 40 26 755 A1 offenbart ein Verbandmaterial auf Folienbasis, das auf der einen Seite mit einem Stützmaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind im Gegensatz zu dem Verbandmaterial gemäß DE 43 14 834 C2 innerhalb der Umfassungsbegrenzung des Trägermaterials angeordnet. Vorzugsweise ist auch hier nur eine Griffleiste auf dem Trägermaterial angebracht.

Im Handel kann dieses Pflaster mit Polyurethanfolie unter dem Namen "Cutifilm" ® ebenfalls von der Firma Beiersdorf bezogen werden.

Bei medizinischen Pflastern, Wundverbänden und Fixierungen aller Art tritt häufig ein Phänomen auf, das zu einer vorzeitigen, unbeabsichtigten Ablösung führt. Dieses Phänomen ist ein Aufrollen des Produkts, meist von einer Ecke oder aber einer Kante des Pflasters ausgehend.

Hat sich das Pflaster an einem Punkt gelöst, folgt eine Kettenreaktion, die sehr rasch zu einem vollständigen Ablösen führt. Besonders oft findet dieses Aufrollen bei Pflastern statt, die unter Kleidung oder in Schuhen getragen werden. Ursache ist die Reibung des Kleidungsstücks oder Schuhs an der Oberfläche des Pflasters. Diese Reibungskraft verursacht eine dynamische Scherbelastung der Haftklebemasse, welche meist sehr schnell zu einem Bruch der Verklebung im Randbereich führt. Nach dem Lösen der Klebmasse an einer Kante haftet das Textil oder Leder an der abstehenden Masse und verursacht durch die tangential anliegende Kraft ein Aufrollen und weiteres, beschleunigtes Ablösen des gesamten Pflasters.

Eine Möglichkeit zur Vermeidung des vorzeitigen Ablösens ist die Erhöhung der Adhäsion der Haftklebemasse auf der Haut. Diese Klebrigkeit läßt sich aber nicht beliebig erhöhen, da es sonst beim beabsichtigten Ablösen des Produkts zu Hautreizungen, Schmerzempfinden und Störung der Wundruhe kommen kann.

Aus der EP 0 409 587 A1 ist bekannt, wie stark das vorzeitige Ablösen von Pflastern von der Kontaktfläche A der Trägerfolie, also jener Fläche, auf der sich zwei gleitende Körper tatsächlich berühren, beeinflußt wird.

Dort wird die Verwendung thermoplastischer Folien beschrieben, die beim oder nach dem Extrudieren in geschmolzenem Zustand durch eine Prägewalze hohlgeprägt werden. Beste Ergebnisse werden mit einer Struktur erhalten, bei der die Kontaktfläche ca. 25% der Gesamtfläche darstellt.

Verschiedene Oberflächen können nach diesem Verfahren nur durch Einsatz unterschiedlicher Prägewalzen erzeugt werden. Zu diesem Zweck müssen die entsprechenden Walzen erst in aufwendigen und teuren Prozessen hergestellt werden. Ein weiterer Nachteil des Verfahrens ist die Erzeugung von Positiv-/Negativ-Strukturen auf beiden Seiten des Trägers. Diese Struktur stellt hohe Anforderungen bei der partiellen Beschichtung mit Klebmasse und der Plazierung und Verankerung von Wundauflagen.

Eine Herstellung von mehrschichtigen Folien aus harten und weichen Ausgangsmaterialien wird nicht beschrieben.

Die Verwendung von mehrschichtigen Folien zur Bildung von Pflastern, hergestellt durch Ausstreichen von Polymer-Dispersionen oder -Lösungen auf geprägten Papieren oder Folien, ist nicht Gegenstand der Offenbarung.

EP 0 446 431 schildert, wie unter Verwendung von Gießpapieren (Casting Papieren) aus Polymer-Lösungen Trägermaterialien für medizinische Zwecke hergestellt werden können. Gegenstand dieser Erfindung ist jedoch ein Laminat aus polymeren Filmschichten und einem makroporösen textilen Material. Das gemäß einem Beispiel verwendete Gießpapier wird nicht näher beschrieben. Auch die Verbesserung von Gleiteigenschaften durch Optimierung der Kontaktfläche und der Elastizität der Deckschicht, erzielt durch Einsatz von Gießpapieren mit strukturierter Oberfläche und Ausgangsmaterialien verschiedener Härte, ist nicht gezeigt.

US 5,643,187 beschreibt ein Filmpflaster, dessen Trägerfilm aus zwei Schichten besteht, wobei die äußere Schicht des Films verhältnismäßig hart, dünn und gleitfähig und die innere Schicht relativ weich und dick ist. Durch diese Kombination zweier unterschiedlicher Materialien soll ein Filmträger mit verbesserten Gleiteigenschaften und ausreichender Stabilität erhalten werden. Diese Schrift schildert ausschließlich die Verwendung von Filmträgern mit glatten, unstrukturierten Oberflächen. Die Verbesserung von Gleiteigenschaften durch Optimierung der Kontaktfläche ist nicht Gegenstand der Beschreibung. Nachteil dieser Methode zur Verbesserung der Gleitfähigkeit ist eine unvermeidliche Einbuße von Dehnbarkeit, Elastizität und damit Anschmiegsamkeit der Trägerfolie. Zur Dehnung von harten Folien oder Folienschichten muß deutlich mehr Kraft aufgebracht werden, so daß es bei der Anwendung als Pflaster zu Hautirritationen und verzögertem Heilungsverlauf aufgrund mechanischer Belastung der Wunde kommen kann.

Aus DE 197 06 380 ist bekannt, daß durch mehrschichtigen Aufbau eine Folie mit richtungsabhängiger Wasserdampfdurchlässigkeit hergestellt werden kann. Durch Coextrusion von thermoplastischen Polyurethanen mit unterschiedlichen Wasserdampfdurchlässigkeiten wird eine mehrschichtige Folie erhalten, die ihrerseits unterschiedliche Durchlässigkeiten für Wasserdampf zeigt, je nachdem, welche Seite des Films der Feuchtigkeitsquelle zugewandt ist. Die Erfindung beschreibt ebenfalls die Verwendung von Polyurethanschichten, die sich neben der Wasserdampfdurchlässigkeit auch in ihrer Härte unterscheiden können, eine Optimierung von Gleiteigenschaften durch Variation der Kontaktfläche der Filme ist aber nicht dargelegt.

WO 98/41590 beschreibt die Herstellung von Filmen auf Basis von Polystyrol- Blockcopolymer/Polystrol-Mischungen durch Schmelzextrusion, welche sich durch gute Reißbarkeit und einfache Darreichung in einer Spenderpackung auszeichnen. Herstellung und Eigenschaften, insbesondere Gleit- und Dehnungseigenschaften, von Folien aus mehreren Schichten unterschiedlicher Härten sind nicht Gegenstand der gesamten Offenbarung.

Die Reibungskraft $F_r$ ist nach dem ersten Gesetz der Reibung gleich dem Produkt aus Reibungskoeffizient $\mu$ und Normalkraft $F_n$. Dieser Koeffizient ist ein Maß für die Kraft, die man aufwenden muß, um einen Körper auf einer Oberfläche zu bewegen, wobei $\mu_s$ den statischen (Haftreibung) und $\mu_k$ den kinetischen (Gleitreibung) Reibungskoeffizienten bezeichnet.

Die Entwicklung von Trägern mit gutem Gleitvermögen, d.h. niedrigen Reibungskoeffizienten, ist demnach ein zentraler Ansatzpunkt, um den oben geschilderten Effekt des Aufrollens zu vermeiden. Obwohl, wie vor allem von Ludema (Ludema, K.C., Friction, Wear, Lubrication: a Textbook in Tribology, CRC Press, Boca Raton 1996) dargestellt, bisher weder exakte noch näherungsweise Methoden existieren, um Reibungs- oder Abnutzungsverhalten aus grundlegenden Prinzipien abzuleiten, erlaubt eine Durchsicht der Literatur Rückschlüsse auf Parameter, welche die Größe von $\mu_s$ und $\mu_k$ bestimmen.

Für die Haftreibung gilt (Blau, PJ.; Friction Science and Technology, Marcel Dekker, New York 1996) folgender Ausdruck:

$$\mu_s = (\tau_m/P^*)\, A$$

wobei

$\tau_m$ die Scherfestigkeit,
A die Kontaktfläche und
P* die Kombination von Normalkraft und Adhäsion

bezeichnet.

Die Gleitreibung zwischen zwei Körpern wird von einer Reihe von Effekten bestimmt, die zusammenwirken (Bhushan, B., Gupta, B.K.; Handbook of Tribology, McGraw-Hill New York 1991). Neben Adhäsionskomponenten treten Plowing-, Rauhigkeits-, Deformations- und, besonders bei viskoelastischen Materialien, Dämpfungseffekte auf. Der relative Beitrag dieser Effekte hängt von den beteiligten Materialien, der Oberflächentopographie, dem Zustand der gleitenden Oberflächen und den Umgebungsbedingungen ab.

Aus Untersuchungen von Bartenev (Bartenev, G.M., Lavrentev, V.V.; Friction and Wear of Polymers, Elsevier Amsterdam 1981) und Rabinowicz (Rabinowicz, E.; Friction and Wear of Materials, Wiley-Interscience, New York 1995) geht hervor, daß neben der Kontaktfläche Parameter wie Rauhigkeit, Härte, Elastizitätsmodul und Oberflächenenergie der Materialien den Reibungskoeffizienten $\mu_k$ bestimmen.

Die Härte eines Kunststoffs wird im allgemeinen als Shore-Härte angegeben. Nach DIN 53505 1987-06 entspricht die Shore-Härte bei der Prüfung von Elastomeren, Gummi und Kautschuk dem Widerstand gegen das Eindringen eines Kegelstumpfes (A oder C) beziehungsweise eines abgerundeten Kegels (D), der durch das Zusammendrücken einer Feder mit einer festgelegten Federcharakteristik gemessen und in dimensionslosen Shore-A(C, D)-Härteeinheiten ausgedrückt wird. Bei der Prüfung von Stahl wird die Shore-Rückprallhärte in dem sogenannten Skleroskop gemessen, bei dem die Rückprallhöhe eines Fallhammers, der in einem senkrechten Rohr auf die Prüffläche fällt, bestimmt wird. Dabei handelt es sich eher um eine Elastizitätsprüfung, weshalb die Meßwerte mit denen der Härteprüfung nach Brinell oder Vickers nur bedingt vergleichbar sind (Römpp Lexikon Chemie - Version 1.5, Stuttgart/New York: Georg Thieme Verlag 1998).

Wie aus obigen Gleichungen abgeleitet werden kann, besteht eine direkte Korrelation zwischen Härte und Gleitfähigkeit eines Materials.

Für die Anschmiegsamkeit und den Tragekomfort eines Filmpflasters ist hingegen die Weichheit, Dehnbarkeit

EP 1 187 586 B1

und Elastizität des Trägerfilms von zentraler Bedeutung. Werden während des Tragens auf der Haut zu hohe Kräfte zur Dehnung des Trägermaterials benötigt, verursacht durch Körperbewegungen wie Beugen von Gelenken oder Anspannung von Muskeln, führen diese hohen Kräfte zu Spannungsgefühl und Hautreizungen. Als geeignete Größe zur Bestimmung der Anschmiegsamkeit eines Filmträgers während der Anwendung auf der Haut hat sich das 100%-Modul erwiesen. Dieser Wert gibt an, welche Zugspannung aufgebracht werden muß, um den Film um 100% zu dehnen, und kann nach DIN 53 504 ermittelt werden.

Aufgabe der Erfindung ist es, die aus dem Stand der Technik bekannten Nachteile zu vermeiden und ein Filmpflaster zur Verfügung zu stellen, das einseitig selbstklebend ausgerüstet ist, eine gute Elastizität und Dehnbarkeit aufweist und das sich aufgrund verbesserter Gleiteigenschaften nicht unbeabsichtigt von der Hautstelle löst, auf der es zuvor verklebt worden ist.

Gelöst wird diese Aufgabe durch ein Filmpflaster, wie es im Hauptanspruch niedergelegt ist.
Gegenstand der Unteransprüche sind dabei vorteilhafte Weiterbildungen des Filmpflasters.

Demgemäß ist Gegenstand der vorliegenden Erfindung ein Filmpflaster insbesondere zur Abdeckung von Wunden und Verhütung oder Behandlung von Blasen, umfassend einen zweischichtigen elastischen Film, wobei die obere Schicht gegenüber der unteren Schicht eine höhere Härte und geringere Dicke aufweist, wobei die obere Schicht eine erhabene Musterung aufweisend strukturiert ist und wobei die Oberfläche der unteren Schicht mit einer Haftklebemasse beschichtet ist.

In einer ersten bevorzugten Ausführungsform ist zwischen der oberen und der unteren Schicht zumindest eine weitere Schicht vorhanden, die u. a. zur Verbesserung der Keimdichtigkeit dient.

Erfindungsgemäß ist unter der Struktur der oberen Schicht eine erhabene Musterung zu verstehen, so daß die Schicht nicht flächenhaft ausgebildet ist, sondern dreidimensionale Erhebungen beziehungsweise Vertiefungen aufweist. Die obere Schicht kann auch in einer bevorzugten Ausführungsform aus einzelnen diskreten (also voneinander getrennten) Segmenten bestehen.

Das Filmpflaster weist somit eine dreidimensional gestaltete Oberfläche auf, die von der oberen Schicht oder der Deckschicht gebildet ist, die ihrerseits dünner und härter als die untere und als weitere, der Klebemasse zugewandte Schichten ist. Überraschenderweise werden durch Optimierung der Struktur und Härte der Deckschicht Folien mit verbesserter Gleitfähigkeit und guter Anschmiegsamkeit und Elastizität erhalten.

Die Oberflächenstruktur der Folien kann insbesondere durch die Wahl des Casting-Substrats festgelegt werden. Bevorzugt sind Casting-Substrate, welche eine Struktur aufweisen, bei der einzelne Erhebungen durch Vertiefungen vollständig voneinander getrennt sind. Der Schichtaufbau der Folie kann so gesteuert werden, daß ausschließlich diese Erhebungen aus hartem, gleitfähigem Material bestehen. Die Polymerkonzentration in der Dispersion oder Lösung und die Breite des Spalts beim Beschichten des Casting-Substrats bestimmen die Dicke der harten Deckschicht.
Werden stark verdünnte Lösungen oder Dispersionen, eventuell unter Verwendung von Netzmitteln, mit niedriger Spaltbreite verstrichen, erhält man Ausführungen des Films, bei denen selbst die Erhebungen zum Großteil aus weichem Polymer bestehen.
Weitere Schichten des erfindungsgemäßen Filmträgers werden durch Ausstreichen von weichen, elastischen Polymer-Typen erzeugt.

In der vorteilhaften Ausführung ist sichergestellt, daß die harten, gleitfähigen Erhebungen durch weiche, elastische "Täler" voneinander getrennt sind. Für die verbesserte Gleitfähigkeit sorgen diese Erhebungen, während die Elastizität und Flexibilität durch das weiche, flexible Grundmaterial gewährleistet ist.

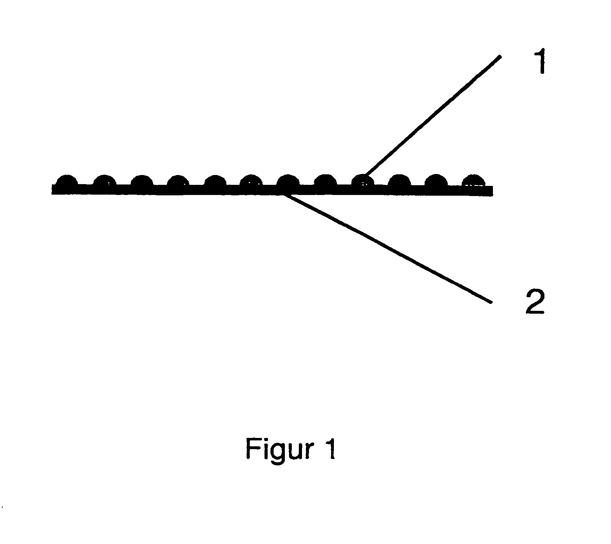

In der Figur 1 ist ein erfindungsgemäßer Film gezeigt, der aus einer harten, gleitfähigen Deckschicht 1 besteht. Die Deckschicht 1 setzt sich aus einzelnen Segmenten zusammen, die vollständig voneinander getrennt sind und die auf einer weichen elastischen Basisschicht 2 aufgebracht sind.

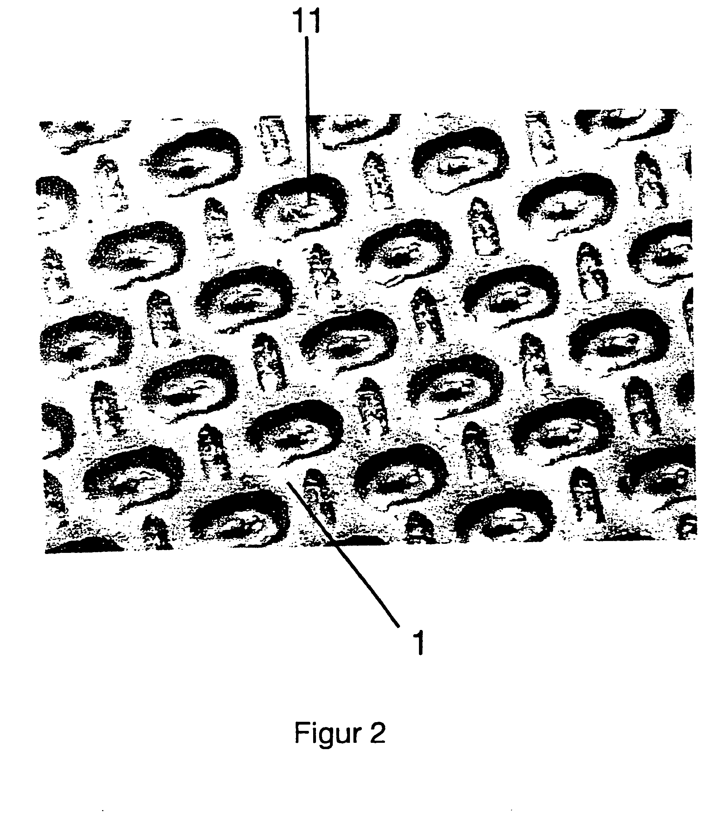

In der Figur 2 ist ein erfindungsgemäßes Filmpflaster in der Draufsicht gezeigt. Die Deckschicht 1 wird von diskreten Segmenten 11 gebildet, die hervorstehen.

Bei den Gieß-Papieren oder Casting-Substraten handelt es sich zumeist um handelsübliche, wasserfeste Silicon- oder Polypropylen-beschichtete Papiere, die hauptsächlich zur Herstellung von Kunstleder eingesetzt werden und in einer Fülle von Varianten zur Verfügung stehen. Durch Wahl des Papiers kann die dreidimensionale Gestalt und damit die Gleiteigenschaften der Folie maßgeblich beeinflußt werden. Als Casting-Substrat ist auch jede andere Folie, jeder Film oder jedes Papier geeignet (beispielsweise Folien aus Polyethylen oder Polypropylen-beschichtete Gießpapiere), welches eine gewünschte Oberflächenstruktur aufweist und durch die eingesetzten Polymer-Dispersionen oder -Lösungen nicht angelöst oder chemisch zersetzt wird.

Casting-Papiere mit der gewünschten Oberflächenstruktur können unter anderem bei den Firmen Arconvert S.p.A. (Arco, Italien), der Firma Binda S.p.A. (Crusinallo/Omegna, Italien) oder Arjo Wiggins Casting Papers Limited (Basing Stoke, England) bezogen werden.

Vorteil der erfindungsgemäßen Pflasterfolie im Gegensatz zum Stand der Technik, wie er insbesondere in der EP 0 409 587 A1 oder der US 5,643,187 dokumentiert ist, ist die optimale Synthese aus Gleitfähigkeit und Dehn-

4

barkeit eines Trägermaterials durch Verwendung einer harten, dreidimensional gestalteten Oberfläche. Bei Prägung eines weichen Films, wie in der EP 0 409 587 A1 dargelegt, werden optimale Gleiteigenschaften nicht erhalten, da weiche Materialien höhere Reibungskoeffizienten aufweisen als harte. Das Aufbringen einer kontinuierlichen, unstrukturierten harten Schicht, wie in US 5,643,187 erläutert, ergibt gute Gleitfähigkeit, verringert aber drastisch die Dehnbarkeit und damit Anschmiegsamkeit des Filmträgers.

Bevorzugte Dispersionen zur Herstellung von medizinischen Pflastern zur Abdeckung von Wunden und Behandlung oder Verhütung von Blasen sind Polyurethan-Dispersionen, welche beispielsweise bei der Bayer AG, Leverkusen, unter dem Namen Impranil und Impraperm erhältlich sind. Diese Dispersionen können durch Zusatz geeigneter Additive aufgeschäumt werden, so daß sich auch Schäume als Träger herstellen lassen. Durch Mischen verschiedener Impranil- und/oder Impraperm-Qualitäten und optionale Herstellung mehrlagiger Schichten durch sukzessives Ausstreichen verschiedener geschäumter oder ungeschäumter Dispersionen auf einem Papier können Trägermaterialien mit gewünschten Eigenschaften wie Härte, Elastizitätsmodul, Dehnbarkeit, Wasserdampfdurchlässigkeit, Rauhigkeit, Griff und Anmutung erzeugt werden. Die an sich farblosen Filme oder Schäume lassen sich durch Zusatz von handelsüblichen Pigmenten wie beispielsweise Euderm (Bayer AG, Leverkusen) einfärben.

Eine bevorzugte Polyurethan-Folie, ausschließlich aus ungeschäumten Schichten bestehend, ist etwa 10 bis 500 μm stark und transparent, eine Polyurethan-Folie, bestehend aus geschäumten und ungeschäumten Schichten, ist etwa 0,1 bis 3 mm stark. Beide Varianten weisen Reißdehnungen von über 450 % und Wasserdampfdurchlässigkeiten von über 500 g/m$^2$ in 24h bei 38 °C und 95 % rel. Feuchte nach DAB auf.

Erfindungsgemäße Filme können auch unter Verwendung von handelsüblichen Polystyrol-Blockcopolymer Compounds erhalten werden. Geeignete Compounds in einem Härtebereich von 29 bis 96 Shore A können beispielsweise von der Firma Gummiwerk Kraiburg, 84464 Waldkraiburg, unter der Bezeichnung Thermolast K, der Firma Silac , La Rochefoucauld, Frankreich, unter der Bezeichnung Lacbloc (SEBS-Compounds), beziehungsweise Terlac (SBS-Compounds) oder der Firma Albis Plastic, Hamburg, unter der Bezeichnung Evoprene G, beziehungsweise Evoprene Super G bezogen werden. Da Polystyrol-Blockcopolymere hervorragende Kompatibilität mit anderen Polymeren aufweisen, können weitere Polymere wie beispielsweise Polystyrol den Lösungen beigemischt werden.

Daneben lassen sich jedoch auch Filme auf Grundlage von Polystyrol-Blockcopolymeren oder anderen bekannten filmbildenden elastischen Polymeren wie beispielsweise Acrylaten verwenden.

Die Dicke der Folien kann dabei zwischen etwa 10 bis 500 μm, vorzugsweise 15 bis 80 μm, das Gewicht entsprechend zwischen etwa 15 bis 350 g/m$^2$, vorzugsweise 15 bis 100 g/m$^2$, die Höchstzugkraft längs zwischen etwa 2 bis 100 N/cm, vorzugsweise 5 bis 40 N/cm, das 100%-Modul weniger als 20 MPa, vorzugsweise weniger als 15 MPa, und die Reißdehnung längs zwischen etwa 100 bis 1000 % betragen.

Dem Fachmann ist ohne weiteres einsichtig, daß erfindungsgemäße Folien auch durch Verwendung von Polymer-Lösungen anstelle von Polymer-Dispersionen hergestellt werden können. Dort kann die Dicke der Deckschicht und Basisschichten über die Konzentration des Polymers in den entsprechenden Polymerlösungen eingestellt werden.

So können durch Wahl des Substrats und der Auswahl, Vorbereitung und Verarbeitung der Rohmaterialien Trägermaterialien mit maßgeschneiderten Gleit- und DehnungsEigenschaften erhalten werden. Dem Fachmann ist unmittelbar einsichtig, daß diese Einflußgrößen auch geeignet sind, Filme mit gewünschter Anmutung und Haptik herzustellen. Für Anwendungen, bei denen weniger die Gleiteigenschaften als die Anmutung im Vordergrund steht, können Eigenschaften wie Anschmiegsamkeit und angenehme Haptik durch gezielte Optimierung der genannten Parameter verbessert werden.

Als Haftklebemassen können handelsübliche Klebmassen medizinischer Qualität eingesetzt werden.

Die Haftklebemasse auf der Folie weist beispielsweise bevorzugt eine Klebkraft auf Stahl von etwa 2 bis 4 N/cm auf, wobei das Prüfmaterial, da die Folie sehr dehnbar ist, für die Messung rückseitig mit einem unelastischen Klebefilm verstärkt werden muß. Die Messung selbst erfolgte in Anlehnung an DAB 9.

Auf seiner selbstklebend ausgerüsteten, später der Haut zugewandten Seite ist das erfindungsgemäße Filmpflaster über seine ganze Breite bis zum Gebrauch üblicherweise mit einem klebstoffabweisenden Trägermaterial, wie silikonisiertem Papier, abgedeckt. Dieses schützt die Selbstklebeschicht aus einer gut hautverträglichen Klebemasse, beispielsweise auf Acrylatbasis, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Das Filmpflaster kann als solches verwendet werden, es kann jedoch auch zusätzlich mittig in geeigneter Breite eine übliche, saugende Wundauflage oder ein anderes funktionales Material, welche positive Auswirkungen auf die Heilung von Wunden oder Blasen haben, aufgebracht sein, so daß es direkt als Wundverband eingesetzt werden kann. Ein derartiger Verband mit Rundum-Verklebung ist besonders vorteilhaft, da er keimdicht und wasserfest ist

Das Produkt kann zur Sterilisation nach Standard-Verfahren verpackt und γ-bestrahlt werden.

Folgende Verfahren haben sich als besonders vorteilhaft für die Herstellung des Filmpflasters herausgestellt.

Zunächst wird eine Polyurethan-Dispersion auf einem geprägten wasserfesten (gegebenenfalls Silicon- oder Polypropylen-beschichteten) Papier oder einer geprägten Folie ausgestrichen, so daß sich eine erhabene Musterung aufweisend strukturierte Schicht ergibt. Der Verbund wird getrocknet.

Eine zweite Polyurethan-Dispersion wird als zweite Schicht auf der ersten ausgestrichen, der Verbund wieder getrocknet.

Der sich daraus ergebende zweischichtige elastische Polyurethanfilm wird mit einer Haftklebemasse beschichtet, die Haftklebemasse gegebenenfalls mit einer Wundabdeckung und einem klebstoffabweisenden Trägermaterial versehen und das wasserfeste Silicon- oder Polypropylen-beschichtete Papier oder die Folie entfernt, wobei die Schicht (1) durch Wahl der Herstellparameter hörter und dünner als die zweite Schicht (2) gewählt wird.

Gegebenenfalls werden zwischen der ersten und der zweiten Schicht weitere Schichten mit den gewünschten Eigenschaften aufgebracht.

Vollflächige oder teilweise Beschichtung der glatten Oberfläche des Trägers mit Haftklebemasse ergibt das medizinische Pflaster.

Die Herstellung der Polystyrol-Blockcopolymer-Trägerfolien umfaßt

das Lösen eines ersten Polystyrol-Blockcopolymer-Compound in einem organischen Lösungsmittel, vorzugsweise Toluol,

Ausstreichen der Lösung auf einem geprägten Kunststoffilm, beispielsweise aus Polyethylen, oder einem Polypropylen-beschichteten Gießpapier, so daß sich eine erhabene Musterung aufweisend strukturierte Schicht ergibt

Trocknen des Verbundes,

Lösen in einem organischen Lösungsmittel und Ausstreichen eines zweiten Polystyrol-Blockcopolymer-Compound als zweite Schicht auf dem ersten,

Trocknen des Verbundes,

Beschichten des sich daraus ergebenden Films mit einer Haftklebemasse,

gegebenenfalls Versehen der Haftklebemasse mit einer Wundabdeckung und einem klebstoffabweisenden Trägermaterial und

Entfernung des Kunststoffilms oder des Gießpapiers wobei die Schicht (1) durch Wahl der Herstellparameter hörter und dünner als die zweite Schicht (2) gewählt wird.

Vorteil des hier erfindungsgemäß beschriebenen Herstellungsverfahrens, welches nicht auf einer Hohlprägung, sondern dem Ausstreichen gelöster oder dispergierter Polymer-Compounds auf Substraten mit strukturierter Oberfläche basiert, ist eine hohe Flexibilität, eine einfache Änderung der Struktur des Trägers durch Wechsel des Substrats, geringe Kosten und eine sehr große Anzahl von verfügbaren Papieren und Folien. Weiterhin wird durch dieses Verfahren eine Oberfläche mit gewünschter Struktur und eine gegenüberliegende, glatte Oberfläche erhalten, welche sich problemlos mit Klebemasse beschichten läßt.

Das erfindungsgemäße Filmpflaster wird nachfolgend in beispielhafter Ausführung beschrieben, ohne damit die Erfindung in irgendeiner Weise beschränken zu wollen.

**Beispiel 1**

Auf dem Gießpapier BNS Cortina (Fa. Binda) wird eine, mit 0,2% Collacral PU85 (BASF AG, Ludwigshafen) verdickte Impranil DLF-Dispersion mittels eines Rakels so ausgestrichen, daß nur die Vertiefungen des Papiers mit Dispersion gefüllt sind.

Nach Trocknen der Deckschicht wird in eine zweite und dritte Schicht, bestehend aus mit 0,25 % Collacral PU85 verdicktem Impranil DLN/DLH 1:1 ausgestrichen.

Nach Trocknen und Entfemen des Gießpapiers wird der weiche, dehnbare Trägerfilm mit strukturierter, harter Deckschicht erhalten.

**Beispiel 2**

Auf dem Gießpapier BNS Cortina (Fa. Binda) wird eine, im Verhältnis 1:4 mit Wasser verdünnte und mit 0,3% des Netzmittels Surfynol 504 (Fa. Biesterfeld, Hamburg) versetzte Impranil DLF-Dispersion mittels eines Rakels so ausgestrichen, daß nur die Vertiefungen des Papiers mit Dispersion gefüllt sind.

Nach Trocknen der Deckschicht wird in eine zweite, dritte und vierte Schicht, bestehend aus mit 0,25 % Collacral PU85 verdicktem Impranil DLN/DLH 1:1, ausgestrichen.

Nach Trocknen und Entfemen des Gießpapiers wird der weiche, dehnbare Trägerfilm mit strukturierter, harter Deckschicht erhalten.

Nachfolgende Tabelle stellt Gleit- und Dehnungseigenschaften erfindungsgemäßer Folien dar, wobei der Reibungskoeffizient μ nach DIN 53375 und das 100%-Modul nach DIN 53504 bestimmt wurden. Die Zusammensetzung der Basisschichten ist zuerst aufgeführt, dann der Aufbau der äußeren Deckschicht. Die Spalte "Substrat" beschreibt das jeweils verwendete Gießpapier. Als Vergleichsbeispiele sind Folien aufgeführt, die aus weichem, dehnbarem Polymer ohne harte, gleitfähige Deckschicht hergestellt wurden.

Tabelle 1:

| Eigenschaften von erfindungsgemäßen Filmen | | | | | |
|---|---|---|---|---|---|
| Nr. | Basisschicht | Deckschicht | Substrat | Modul 100% [MPa] | μ |
| Vergleichsbsp. 1 | DLH/DLN 1:1 | - | AW Pearl | 2,3 | 2,7 |
| 2 | DLH/DLN 1:1 | DLF/PU 85 | AW Pearl | 2,6 | 0,4 |
| 3 | DLH/DLN 1:1 | DLF/Netzmittel | AW Pearl | 3,6 | 0,5 |
| Vergleichsbsp. 2 | DLH/DLN 1:1 | - | BNS Cortina | 1,5 | 1,8 |
| 4 | DLH/DLN 1:1 | DLF/PU 85 | BNS Cortina | 2,3 | 0,4 |
| 5 | DLH/DLN 1:1 | DLF verd. /Netzmittel | BNS Cortina | 1,7 | 0,5 |

Ein Vergleich der Folien mit und ohne harte, strukturierte Deckschicht zeigt deutlich die Vorteile der erfindungsgemäßen Trägermaterialien. Die Reibungskoeffizienten μ nehmen in Gegenwart der harten Deckschichten drastisch ab. Die Anschmiegsamkeit und Dehnbarkeit, gemessen als 100%-Modul, bleibt dagegen weitgehend erhalten. Besonders deutlich zeigt Nr. 5 die Vorteile der erfindungsgemäßen Filme. Hier wurde die harte Deckschicht durch Verdünnen der Polyurethan-Dispersion bei gleichzeitigem Zusatz eines Netzmittels so dünn gestaltet, daß das 100%-Modul praktisch unverändert bleibt

## Patentansprüche

1. Filmpflaster umfassend einen zweischichtigen elastischen Film, wobei die obere Schicht (1) gegenüber der unteren Schicht (2) eine höhere Härte und geringere Dicke aufweist, wobei die obere Schicht (1) eine erhabene Musterung aufweisend strukturiert ist und wobei die Oberfläche der unteren Schicht (2) mit einer Haftklebemasse beschichtet ist.

2. Filmpflaster nach Anspruch 1, **dadurch gekennzeichnet, dass** zwischen der oberen (1) und der unteren Schicht (2) zumindest eine weitere Schicht vorhanden ist.

3. Filmpflaster nach Anspruch 1, **dadurch gekennzeichnet, dass** die obere Schicht (1) aus einzelnen voneinander getrennten Segmenten besteht.

4. Filmpflaster nach Anspruch 1, **dadurch gekennzeichnet, dass** die Schichten aus Polyurethan-Dispersionen, Polystyrol-Blockcopolymeren oder Acrylaten hergestellt werden.

5. Filmpflaster nach Anspruch 1, **dadurch gekennzeichnet, dass** das Filmpflaster über seine ganze Breite bis zum Gebrauch mit einem klebstoffabweisenden Trägermaterial, wie silikonisiertem Papier, abgedeckt ist.

6. Filmpflaster nach Anspruch 1, **dadurch gekennzeichnet, dass** das Filmpflaster mittig in geeigneter Breite eine übliche, saugende Wundauflage oder ein anderes funktionales Material, welches positive Auswirkungen auf die Heilung von Wunden oder Blasen haben kann, aufweist.

7. Verfahren zur Herstellung eines Filmpflasters, wobei
eine Polyurethan-Dispersionen auf einer geprägten Folie oder einem geprägten wasserfesten Papier ausgestrichen wird, so dass sich eine erhabene Musterung aufweisende strukturierte Schicht (1) ergibt,
der Verbund getrocknet wird,
eine zweite Polyurethan-Dispersionen als zweite Schicht (2) auf der ersten ausgestrichen wird,
der Verbund getrocknet wird,
der sich daraus ergebene zweischichtige elastische Polyurethanfilm auf der zweiten Schicht (2) mit einer Haftklebemasse beschichtet wird und
das wasserfeste Papier oder die Folie entfernt wird,
wobei die Schicht (1) durch Wahl der Herstellparameter härter und dünner als die zweite Schicht (2) gewählt wird.

**8.** Verfahren zur Herstellung eines Filmpflasters, wobei

ein erster Polystyrol-Blockcopolymer-Compound in einem organischen Lösungsmittel gelöst wird, vorzugsweise Toluol,

die Lösung auf einem geprägten Kunststofffilm, beispielsweise aus Polyethylen, oder einem Polypropylen-beschichteten Gießpapier ausgestrichen wird, so dass sich eine erhabene Musterung aufweisende strukturierte Schicht (1) ergibt,

der Verbund getrocknet wird,

eine zweiter Polystyrol-Blockcopolymer-Compound in einem organischen Lösungsmittel gelöst wird und als zweite Schicht (2) auf dem ersten ausgestrichen wird,

der Verbund getrocknet wird,

der sich daraus ergebene Film mit einer Haftklebemasse beschichtet wird, die Haftklebemasse gegebenenfalls mit einer Wundabdeckung und einem klebstoffabweisenden Trägermaterial versehen wird und

der Kunststofffilm oder das Gießpapier entfernt wird,

wobei die Schicht (1) durch Wahl der Herstellparameter härter und dünner als die zweite Schicht (2) gewählt wird.

**9.** Verfahren gemäß Anspruch 7 oder 8, **dadurch gekennzeichnet, dass** die obere Schicht (1) so ausgestrichen wird, so dass sich eine strukturierte Schicht aus einzelnen voneinander bestehenden Segmenten ergibt.

**Claims**

**1.** Film plaster comprising a two-layer elastic film, the upper layer (1) having a greater hardness and lower thickness than the bottom layer (2), the upper layer (1) being textured, with a raised patterning, and the surface of the lower layer (2) being coated with a pressure-sensitive adhesive.

**2.** Film plaster according to Claim 1, **characterized in that** between the upper layer (1) and lower layer (2) there is at least one further layer.

**3.** Film plaster according to Claim 1, **characterized in that** the upper layer (1) is composed of individual, separate segments.

**4.** Film plaster according to Claim 1, **characterized in that** the layers are produced from polyurethane dispersions, polystyrene block copolymers or acrylates.

**5.** Film plaster according to Claim 1, **characterized in that** the film plaster is covered over its entire width, up until the time of use, with an anti-adhesive carrier material, such as siliconized paper.

**6.** Film plaster according to Claim 1, **characterized in that** the film plaster comprises centrally in an appropriate width a customary, absorbent wound contact material or another functional material which may have beneficial effects on the healing of wounds or blisters.

**7.** Process for producing a film plaster, in which

a polyurethane dispersion is coated out on an embossed film or an embossed water-resistant paper, to give a textured layer (1) exhibiting a raised patterning,

the assembly is dried,

a second polyurethane dispersion is coated out as a second layer (2) on the first,

the assembly is dried,

the resultant two-layer elastic polyurethane film is coated on the second layer (2) with a pressure-sensitive adhesive, and

the water-resistant paper or the film is removed,

the layer (1) being chosen, through the choice of the production parameters, to be harder and thinner than the second layer (2).

**8.** Process for producing a film plaster, in which

a first polystyrene block copolymer compound is dissolved in an organic solvent, preferably toluene,

the solution is coated out on an embossed polymeric film, made of polyethylene for example, or a polypropylene-coated casting paper, to give a textured layer (1) exhibiting a raised patterning,

the assembly is dried,

a second polystyrene block copolymer compound is dissolved in an organic solvent and is coated out as a second layer (2) on the first,

the assembly is dried,

the resultant film is coated with a pressure-sensitive adhesive,

the pressure-sensitive adhesive is provided if desired with a wound cover and with an anti-adhesive carrier material, and

the polymeric film or casting paper is removed,

the layer (1) being chosen, through the choice of the production parameters, to be harder and thinner than the second layer (2).

9. Process according to Claim 7 or 8, **characterized in that** the upper layer (1) is coated out in such a way as to give a textured layer of individual, separate segments.

**Revendications**

1. Pansement pelliculaire, comprenant un film élastique à deux couches, la couche supérieure (1) présentant, par rapport à la couche inférieure (2), une dureté supérieure et une épaisseur inférieure, la couche supérieure (1) étant structurée en présentant un dessin en relief et la surface de la couche inférieure (2) étant revêtue par une masse auto-adhésive.

2. Pansement pelliculaire selon la revendication 1, **caractérisé en ce qu'**au moins une autre couche se trouve entre la couche supérieure (1) et la couche inférieure (2).

3. Pansement pelliculaire selon la revendication 1, **caractérisé en ce que** la couche supérieure (1) est constituée par différents segments séparés les uns des autres.

4. Pansement pelliculaire selon la revendication 1, **caractérisé en ce que** les couches sont préparées à partir de dispersions de polyuréthane, de copolymères à blocs de polystyrène ou d'acrylates.

5. Pansement pelliculaire selon la revendication 1, **caractérisé en ce que** le pansement pelliculaire est recouvert sur toute sa largeur, jusqu'à l'utilisation, par un matériau support repoussant les adhésifs, tel qu'un papier siliconé.

6. Pansement pelliculaire selon la revendication 1, **caractérisé en ce que** le pansement pelliculaire présente au centre, en une largeur appropriée, un recouvrement de plaie absorbant, usuel ou un autre matériau fonctionnel qui peut présenter des effets positifs sur la guérison de plaies ou de cloches.

7. Procédé pour la fabrication d'un pansement pelliculaire, dans lequel une dispersion de polyuréthane est étalée sur une feuille estampée ou un papier résistant à l'eau, estampé, de manière à obtenir la couche (1) structurée présentant un dessin en relief, le composite est séché, une deuxième dispersion de polyuréthane est étalée comme deuxième couche (2) sur la première, le composite est séché, le film de polyuréthane élastique à deux couches ainsi formé est revêtu sur la deuxième couche (2) par une masse auto-adhésive et le papier résistant à l'eau ou la feuille est enlevé, la couche (1) étant choisie plus dure et plus mince que la couche (2) par le choix des paramètres de fabrication.

8. Procédé pour la préparation d'un pansement pelliculaire, dans lequel on dissout un premier compound de copolymère à blocs de polystyrène dans un solvant organique, de préférence le toluène, on étale la solution sur un film en matériau synthétique estampé, par exemple en polyéthylène, ou un papier moulé revêtu de polypropylène, de manière à obtenir la couche (1) structurée présentant un dessin en relief, le composite est séché, un deuxième compound de copolymères à blocs de polystyrène est dissous dans un solvant organique et étalé comme deuxième couche (2) sur la première, le composite est séché, le film ainsi obtenu est revêtu par une masse auto-adhésive, la masse auto-adhésive est pourvue le cas échéant d'un recouvrement de plaie et d'un matériau support repoussant les adhésifs et le film en matériau synthétique ou le papier moulé est éliminé, la couche (1) étant choisie plus mince et plus dure que la deuxième couche (2) par le choix des paramètres de fabrication.

9. Procédé selon la revendication 7 ou 8, **caractérisé en ce que** la couche supérieure (1) est étalée de manière à obtenir une couche structurée constituée par différents segments séparés les uns des autres.

Figur 1

11

Figur 2